# EUROPEAN PATENT APPLICATION

(11) **EP 0 815 851 A2**
(43) Date of publication of application: **07.01.1998**
(21) Application number: 97110516.8
(22) Date of filing: 26.06.1997
(51) Int. Cl.: A61K 9/08, A61K 7/075, A61K 7/16, A61K 9/00, A61K 7/50

(54) **Method of reducing irritativeness of surface active agents and aqueous compositions with reduced irritativeness**

(30) Priority: 27.06.1996 JP 188159/96; 12.09.1996 JP 265151/96
(71) Applicant: Senju Pharmaceutical Co., Ltd., Chuo-ku, Osaka (JP)
(72) Inventor: Doi, Koji, Kobe-shi, Hyogo-ken (JP); Aki, Hiroshi, Kobe-shi, Hyogo-ken (JP); Isaka, Miyoko, Kobe-shi, Hyogo-ken (JP); Sakaki, Hideyuki, Kobe-shi, Hyogo-ken (JP); Kimura, Masako, Kakogawa-shi, Hyogo-ken (JP)
(74) Representative: Kinzebach, Werner, Dr.

(57) **Abstract**

A method to substantially eliminate irritativeness caused by surface active agents contained in an aqueous composition is provided. The method employs, in the production of said composition, in combination one or more surface active agents having an anionic active group with one or more nonionic surface active agents, with a mutual ratio of said one or more surface active agents having an anionic active group to said one or more nonionic surface active agents being 1:1 to 1:6 in concentration by weight. Aqueous compositions with reduced irritativeness as well as a method of solubilization of an insoluble or scarcely soluble pharmaceutical ingredient in producing an external-use aqueous pharmaceutical preparation are also provided.

## Description

### Field of the Invention

The present invention relates to a method of reducing irritativeness, in particular irritativeness to mucous membranes, of surface active agents contained in an aqueous composition, and to aqueous compositions containing surface active agents in which irritativeness of each of the surface active agents is reduced in the composition. The present invention further relates to a method of solubilization , i.e. a method to dissolve or homogeneously disperse insoluble or scarcely soluble drugs in water with the aid of surface active agents in which method the surface active agents are used in the manner to reduce their irritativeness to mucous membranes. The present invention also relates to aqueous compositions thus produced with reduced irritativeness to mucous membranes.

### Background of the Invention

It is known, in producing aqueous preparations for external use such as eye drops, nasal drops, ear drop, to employ surface active agents as solubilizers (which include "emulsifiers" and "suspension aids" in this specification) to dissolve, or to aid to homogeneously disperse, as emulsion or suspension if not full dissolution, insoluble or scarcely soluble drugs in water. Surface active agents typically used for this purpose are nonionic surface active agents including polyoxyethylene sorbitan mono-oleate (Tween, polysorbate), polyoxyethylenehydrogenated castor oil, polyoxyethylene monostearyl. It is because of their relatively low irritativeness to mucous membrane s and the cornea that nonionic surface active agents have been and are widely used so far. However, they do not always provide enough solubilizing effect required. Theoretically, their use at high concentrations would will successfully enhance their solubilizing effect, but it would also increase their irritativeness to such a level that their use in pharmaceutical preparations could not be tolerated. Thus, for ophthalmologic and otorhinologic preparations, these nonionic surface active agents have been used below their respective actual upper limit concentrations to keep their irritativeness to mucous membranes negligible. Such concentrations are, for example, 0.5 W/V% for polyoxyethylene sorbitan mono-oleate 80 (polysorbate 80), 0.5 W/V% for polyoxyethylenehydrogenated caster oil 60 (HCO-60), 0.08 W/V% for polyoxyethylene monostearyl. With these concentrations, however, the number of insoluble or scarcely soluble drugs is limited which can be dissolved or homogeneously dispersed in water. As a result, there are a number of insoluble or scarcely soluble drugs which cannot be dissolved or homogeneously dispersed in water up to concentrations necessary to exhibit their pharmacological effect, even with the aid of actual upper limit concentrations of these nonionic surface active agents.

On the other hand, anionic surface active agents, which are widely used in other fields, have more potent solubilizing activity and could therefore be ideal if usable for this purpose in preparing external-use aqueous pharmaceutical preparations. However, as they are accompanied by much stronger irritativeness to mucous membranes thereby exhibiting severe irritation even at low concentrations, they could not be used in certain types of external-use aqueous pharmaceutical preparations which are applied to, or have possibility to be brought into contact with, highly sensitive tissues including mucous membranes, e.g. eye drops, nasal drops or ear drops (which could be transferred to the mucous membrane side when a perforation is present in the tympanic membrane). In addition, ampholytic surface active agents, i.e. surface active agents having a cationic active group and an anionic active group in the same molecule, also have not been used in such types of external-use aqueous pharmaceutical preparations which are applied to, or have possibility to be brought into contact with, highly sensitive tissues including mucous membranes.

When a pharmacologically effective, intended ingredient of external-use aqueous pharmaceutical preparations such as ophthalmologic or otorhinologic preparations are insoluble or scarcely soluble in a base solution, so that it will be separated from the aqueous phase without dissolving nor forming a stable, homogeneously dispersed mixture system, then employment of such a ingredient is not preferable, considering the difficulty in assuring constant dosage per application as well as from the the viewpoint of quality control of the product in manufacturing facilities. There are a number of clinically useful drugs which are insoluble or scarcely soluble in water at pH ranges physiologically acceptable to application sites for , e.g. hose for ophthalmologic topical application or intranasal application. On the other hand, due to irritativeness to highly sensitive tissues such as mucous membranes, surface active agents so far have failed to offer adequate solubilizing effect. Thus, a number of such drugs considered to be potentially useful for external-use pharmaceutical preparations in the fields of ophthalmology and otorhinolaryngology have been barred from application to pharmaceutical preparations (such as eye drops and nasal drops) applied to highly sensitive tissues.

### Summary of the Invention

Upon this background, it is an objective of the present invention to provide a method of reducing irritativeness to mucous membranes or to the cornea caused by surface active agents contained in an aqueous composition.

It is another objective of the present invention to provide aqueous compositions containing surface active agents with reduced irritativeness to mucous membranes and to the cornea.

It is still another objective of the present invention to provide a method of solubilization of an insoluble or scarcely soluble pharmaceutical ingredient in producing an external-use aqueous pharmaceutical preparation without causing irritativeness to mucous membranes and to the cornea.

Specifically, it is also an objective of the present invention to provide a means to substantially eliminate irritativeness of aqueous compositions to be applied to, or having possibility through normal or erroneous use to be brought into contact with, mucous membranes.

The present inventors have discovered that mixing of one or more surface active agents having an anionic active group (including anionic surface active agents and ampholytic surface active agents) and one or more nonionic surface active agents at a certain mutual ratio in water almost totally eliminates the irritativeness of those surface active agents.

Therefore, the present invention provides a method of reducing irritativeness caused by surface active agents contained in an aqueous composition, by employing in combination, in the production of said composition, one or more surface active agents having an anionic active group with one or more nonionic surface active agents.

The present invention also provides aqueous compositions with reduced irritativeness to mucous membranes and to the cornea containing one or more surface active agents having an anionic active group together with one or more nonionic surface active agents.

In addition, the present invention further provides a method of solubilization of an insoluble or scarcely soluble pharmaceutical ingredient in producing an external-use aqueous pharmaceutical preparation, comprising a step of mixing said pharmaceutical ingredient with an aqueous solution containing one or more surface active agents having an anionic active group and one or more nonionic surface active agents.

In this invention, the term "aqueous compositions" refers to compositions such as aqueous solution, suspension, emulsion, paste and the like, including shampoo, toothpaste, contact lens care preparation such as contact lens cleaner, as well as eye drops, nasal drops, ear drops, dermatologic preparation, preparation for the mouth such as medicinal toothpaste, and other external-use aqueous pharmaceutical preparations such as vaginal washing preparation, etc.

In the specification, the term "external-use aqueous pharmaceutical preparations" refers to aqueous solution, suspension, emulsion, paste and the like which are applied externally to the body through a route other than oral or parenteral one, and the sites of application thereof include the skin, its traumatic area, oral cavity, the cornea and conjunctiva, nasal cavity, vagina and the like.

### Detailed Description of the Invention

Surface active agents having an anionic active group have very strong irritativeness to mucous membranes and to the cornea even at low concentrations, so that they have not been used for pharmaceutical preparations such as eye drops or nasal drops. Nonionic surface active agents also become too irritative for use above certain concentration levels (for example, 0.5 W/V% for polysorbate 80 in eye drops). However, the present inventors discovered that aqueous solutions containing both of them at a certain mutual ratio are almost totally removed of irritativeness even at very high concentrations (for example, 1 W/V% sodium lauryl sulfate + 5 W/V% POE (25) lauryl ether). It was also confirmed that this phenomenon is not limited to a combination of a specific nonionic surface active agent and a specific surface active agent having an anionic active group, but also equally observed in a variety of combinations of surface active agents having an anionic active group and nonionic surface active agents. It was also confirmed that this effect is observed even when the ratio of a surface active agent having an anionic active group to a nonionic surface active agent is 1:1, and becomes more notable at a ratio of 1:2, and even more remarkable at a ratio of 1:4-1:6.

This phenomenon, that a non irritative composition having solubilizing effect is obtained from surface active agents having an anionic active group (which are too irritative to be tolerable) and high concentrations of nonionic surface active agents (which then exhibit strong irritativeness) combined in water at a certain mutual ratio, is unexpectable from ordinary knowledge in the field of pertaining art. The mechanism of this phenomenon has not been clarified so far.

Together with one or more surface active agents having an anionic active group and one or more nonionic surface active agents as solubilizers, the external-use aqueous pharmaceutical composition of the invention may contain other pharmaceutical ingredients, e.g. pharmacologically active compounds, pH adjusting agents, buffering agents, osmotic pressure adjusting agents, suspension aides, antioxidants, preservatives and the like.

Examples of external-use aqueous pharmaceutical preparations of the present invention include, but not limited to, eye drops, nasal drops, ear drops and dermatologic preparations. The combination of the surface active agents in preparations of the present invention makes it possible to provide insoluble or scarcely soluble drugs in the form of homogeneous aqueous pharmaceutical preparations, preferably in the form of aqueous solutions causing no substantial irritativeness to mucous membranes or the cornea.

In the specification, the term "insoluble or scarcely soluble pharmaceutical ingredient" refers to an ingredient of an external-use aqueous pharmaceutical preparation which is difficult to dissolve or homogeneously disperse in the preparation up to a desired concentration, and includes insoluble or scarcely soluble pharmacologically active compounds as well as scarcely soluble complexes formed by more than one ingredients in the preparation.

In the above-described composition and method, the mutual ratio of one or more surface active agents having an anionic active group to one or more nonionic surface active agents is preferably 1: 1 to 1:6 by weight concentration, more preferably 1:2 to 1:6, and still more preferably 1:4 to 1:6.

According to the present invention, aqueous compositions containing one or more surface active agents having an anionic active group and one or more nonionic surface active agents at a certain mutual ratio have substantially no irritativeness to mucous membranes and the cornea, even when their respective concentration is unexpectably high in light of ordinary knowledge in the pertaining art. For example, no irritativeness is observed even when containing as high as 1 % of a surface active agent having an anionic active group (so far actually unusable due to irritativeness) and 5 W/V% of a nonionic surface active agent (which is ten times the actual upper limit so far, i.e. 0.5 W/V%). Moreover, no substantial irritativeness is observed when containing 2 W/V% of a surface active agent having an anionic active group and 10 W/V% of a nonionic surface active agent.

Among surface active agents having an anionic active group, examples of anionic surface active agents usable in the invention include, but not limited to, sulfonic acid salts and salts of sulfonic acid esters such as sodium lauryl sulfate; carbonic acid salts such as sodium N-lauroyl sarcosinate (Sarcosinate LN: NIKKOL); and salts of phosphate esters such as Hosten HLP-N and the like.

Among surface active agents having an anionic active group, examples of ampholytic surface active agents usable in the invention include, but not limited to, lauryldimethylaminoacetic acid betaine.

Examples of nonionic surface active agents include, but not limited to; polyoxyethylene-based surface active agents such as polyoxyethylenehydrogenated castor oil (e.g., HCO-60), polyoxyethylenesorbitan monooleate (e.g., Tween 80), polyoxyethylene lauryl ether (e.g., POE(25) lauryl ether), and polyoxyethylene monostearate (e.g., POE(40) monostearate); polyalcohol esters such as decaglyceryl monolaurate (Decaglyn 1-L; NIKKOL); ethyleneoxide/propyleneoxide block copolymers such as polyoxyethylene polyoxypropylene glycol (e.g., Pronon 102: NOF Corporation).

As the present invention enables to use high concentrations of surface active agents, in particular, surface active agents having an anionic active group so far unusable, without causing irritation, it make it possible to dissolve or homogeneously disperse insoluble or scarcely soluble pharmacologically active compounds which have been unable to use for external-use aqueous pharmaceutical preparations, thereby providing new aqueous pharmaceutical preparations containing such compounds.

In addition, as the present invention enables to suppress irritativeness of surface active agents having an anionic active group (which have strong cleansing power), the combination of surface active agents according to the present invention may, besides the use for the purpose of solubilization as mentioned above, be used to suppress irritativeness of, and thereby improve the safety of, compositions to be, or having possibility to be, brought into contact with mucous membranes or the cornea (e.g., toothpaste and shampoo) and other variety of aqueous compositions containing surface active agents and having possibility to be brought into contact with mucous membranes when erroneously used, such as contact lens care preparations (e.g., contact lens cleaners).

### Animal Tests

Some examples of animal tests will be presented below to demonstrate the effect of the present invention, i.e. elimination of irritativeness by combining surface active agents having an anionic surface active group and nonionic surface active agents.

### Anterior Ocular Irritation Test in Rabbit - 1

### Animals and topical application procedures:

The following composition of formulations 1 -3 were instilled into the eyes of 8 to 9-week old male Japanese white rabbits (Groups 1 to 3, respectively; three animals/group), and evaluated for the presence and extent of irritation to the anterior segment of the eye. Topical application was carried out to one eye (tested eye) with two drops a time, 8 times at one hour intervals for formulations 1 and 2, and to the opposite eye (control eye) was simultaneously instilled physiological saline. For formulation 3, instillation was carried out to one eye (tested eye) only once, and the opposite eye was simultaneously instilled physiological saline.

### Method for Observation and Evaluation:

For Groups 1 and 2, gross observation, i.e. visual examination of the anterior segment of the eye was performed immediately before the initiation of topical application, thirty minutes after each of the first, second, third, forth, sixth and eighth applications, and on the following day (i.e., 24 hours later), and slitlamp microscopy for fluorescein staining on the cornea was performed immediately before the initiation of the application and thirty minutes after the last application. For Group 3, gross observation was performed immediately before and 0.5, 1, 1.5, 2, 2.5, 3, 5 and 24 hours after the application, and slitlamp microscopy for fluoresceine staining on the cornea was performed immediately before and 5 hours after the application. Modified Draize test was employed for scoring and evaluation in gross observation.

| Formulation 1: | |
|---|---|
| Sodium lauryl sulfate | 0.6 g |
| Polysorbate 80 | 3.0 g |
| Boric acid | 1.8 g |
| Benzalkonium chloride | 0.01 g |
| Hydrochloric acid | q.s. (pH 6.7) |
| Distilled water | q.s. |
| Total amount | 100 mL |

| Formulation 2: | |
|---|---|
| Sodium lauryl sulfate | 0.5 g |
| POE(40) monostearate | 2.5 g |
| Boric acid | 1.8 g |
| Benzalkonium chloride | 0.01 g |
| Hydrochloric acid | q.s. (pH 6.7) |
| Distilled water | q.s. |
| Total amount | 100 mL |

| Formulation 3: | |
|---|---|
| Sodium lauryl sulfate | 0.5 g |
| Sodium chloride | 0.9 g |
| Hydrochloric acid | q.s. (pH 6.7) |
| Distilled water | q.s. |
| Total amount | 100 mL |

### Results:

### (1) Gross Observation

The results are presented in Tables 1 to 6. For the cornea and iris, figures are omitted for their scores were all "0".

**TABLE 1**

| Scores for Formulation 1 applied eyes of Group 1 | | | | | | | | |
|---|---|---|---|---|---|---|---|---|
| Observ. time | Ini. | 1st | 2nd | 3rd | 4th | 6th | 8th | Fin. |
| Redness of palpebral conjunctiva | 0 | 0.17 | 0.17 | 0.33 | 0.33 | 0.17 | 0.50 | 0 |
| Edema of palpebral conjunctiva | 0 | 0 | 0 | 0 | 0 | 0 | 0 | 0 |
| Redness of bulbar conjunctiva | 0 | 0.33 | 0 | 0.17 | 0.17 | 0.17 | 0.17 | 0 |
| Nictitating membrane | 0 | 0 | 0 | 0 | 0 | 0 | 0 | 0 |
| Discharge | 0 | 0 | 0 | 0 | 0.33 | 0.33 | 0.33 | 0 |
| Total score | 0 | 0.50 | 0.17 | 0.50 | 0.83 | 0.67 | 1.00 | 0 |

**TABLE 2**

| Scores for physiological saline applied eyes of Group 1 | | | | | | | | |
|---|---|---|---|---|---|---|---|---|
| Observ. time | Ini. | 1st | 2nd | 3rd | 4th | 6th | 8th | Fin. |
| Redness of palpebral conjunctiva | 0 | 0 | 0.17 | 0.17 | 0.33 | 0.17 | 0 | 0 |
| Edema of palpebral conjunctiva | 0 | 0 | 0 | 0 | 0 | 0 | 0 | 0 |
| Redness of bulbar conjunctiva | 0 | 0.17 | 0 | 0 | 0 | 0 | 0 | 0 |
| Nictitating membrane | 0 | 0 | 0 | 0 | 0 | 0 | 0 | 0 |
| Discharge | 0 | 0 | 0 | 0 | 0 | 0 | 0 | 0 |
| Total score | 0 | 0.17 | 0.17 | 0.17 | 0.33 | 0.17 | 0 | 0 |

**TABLE 3**

| Scores for Formulation 2 applied eyes of Group 2 | | | | | | | | |
|---|---|---|---|---|---|---|---|---|
| Observ. time | Ini. | 1st | 2nd | 3rd | 4th | 6th | 8th | Fin. |
| Redness of palpebral conjunctiva | 0 | 0.67 | 0.50 | 0.33 | 0.33 | 0.17 | 0.50 | 0 |
| Edema of palpebral conjunctiva | 0 | 0 | 0 | 0 | 0 | 0 | 0 | 0 |
| Redness of bulbar conjunctiva | 0 | 0.33 | 0 | 0 | 0 | 0 | 0.17 | 0 |
| Nictitating membrane | 0 | 0 | 0 | 0 | 0 | 0 | 0 | 0 |
| Discharge | 0 | 0 | 0 | 0 | 0 | 0.33 | 0.33 | 0 |
| Total score | 0 | 1.00 | 0.50 | 0.33 | 0.33 | 0.50 | 1.00 | 0 |

**TABLE 4**

| Scores for physiological saline applied eyes of Group 2 | | | | | | | | |
|---|---|---|---|---|---|---|---|---|
| Observ. time | Ini. | 1st | 2nd | 3rd | 4th | 6th | 8th | Fin. |
| Redness of palpebral conjunctiva | 0 | 0.33 | 0.17 | 0.17 | 0.17 | 0.17 | 0.17 | 0 |
| Edema of palpebral conjunctiva | 0 | 0 | 0 | 0 | 0 | 0 | 0 | 0 |
| Redness of bulbar conjunctiva | 0 | 0 | 0 | 0 | 0 | 0 | 0.17 | 0 |
| Nictitating membrane | 0 | 0 | 0 | 0 | 0 | 0 | 0 | 0 |
| Discharge | 0 | 0 | 0 | 0 | 0 | 0 | 0 | 0 |
| Total score | 0 | 0.33 | 0.17 | 0.17 | 0.17 | 0.17 | 0.34 | 0 |

**TABLE 5**

| Scores for Formulation 3 applied eyes of Group 3 | | | | | | | | | |
|---|---|---|---|---|---|---|---|---|---|
| hours | Ini. | 0.5 | 1 | 1.5 | 2 | 2.5 | 3 | 5 | 24 |
| Redness of palpebral conjunctiva | 0 | 1.00 | 1.67 | 1.00 | 1.00 | 1.00 | 0.83 | 0.50 | 0 |
| Edema of palpebral conjunctiva | 0 | 0.33 | 0.50 | 0.83 | 0.83 | 0.83 | 0.17 | 0.17 | 0 |
| Redness of bulbar conjunctiva | 0 | 0.83 | 0.83 | 0.67 | 0.50 | 0.67 | 0.50 | 0.33 | 0 |
| Nictitating membrane | 0 | 0.67 | 0.67 | 0.67 | 0.33 | 0.50 | 0.50 | 0.50 | 0 |
| Discharge | 0 | 1.00 | 1.00 | 1.00 | 1.33 | 1.00 | 1.00 | 0 | 0 |
| Total score | 0 | 3.83 | 4..67 | 4.17 | 4.00 | 4.00 | 3.00 | 1.50 | 0 |

**TABLE 6**

| Scores for physiological saline applied eyes of Group 3 | | | | | | | | | |
|---|---|---|---|---|---|---|---|---|---|
| hours | Ini. | 0.5 | 1 | 1.5 | 2 | 2.5 | 3 | 5 | 24 |
| Redness of palpebral conjunctiva | 0 | 0 | 0 | 0.17 | 0 | 0 | 0 | 0 | 0 |
| Edema of palpebral conjunctiva | 0 | 0 | 0 | 0 | 0 | 0 | 0 | 0 | 0 |
| Redness of bulbar conjunctiva | 0 | 0.17 | 0 | 0 | 0 | 0 | 0 | 0 | 0 |
| Nictitating membrane | 0 | 0.67 | 0.67 | 0.67 | 0.33 | 0.50 | 0.50 | 0.50 | 0 |
| Discharge | 0 | 0 | 0 | 0 | 0 | 0 | 0 | 0 | 0 |
| Total score | 0 | 0.17 | 0 | 0.17 | 0 | 0 | 0 | 0 | 0 |

As evident from the tables, the findings of anterior segment of the eyes topically received Formulation 1 or 2 were nearly comparable to the eyes received physiological saline as a control. On the other hand, with Formulation 3 (formulation for comparison), redness of palpebral conjunctiva, bulbar conjunctiva and nictitating membrane as well as discharge were noted, and two eyes out of the three exhibited edema of conjunctiva. In the eye with severe edema of palpebral conjunctiva, the edema covered the bulbar conjunctiva and thereby rendering it impossible to conduct evaluation of the redness of bulbar conjunctiva. Thus, evaluation of redness of bulbar conjunctiva was conducted only for eyes without severe edema. Therefore, the figures for redness of bulbar conjunctiva in Table 5 are considered to be smaller than the degree of actual occurring redness. These changes reached their peak at one hour and was observed to continue up to three hours after the topical application of Formulation 3. After five hours of topical application, a trend to recovery was noted, and full recovery was observed the following day.

### (2) Findings in corneal fluorescein staining:

No abnormality was noted in any group.

### Conclusion:

As evident from the results, for the formulation for comparison (Formulation 3), which contains anionic surface active agent sodium lauryl sulfate as a sole surface active agent, a single topical application exhibited a high degree of irritativeness to the conjunctiva. In contrast, solutions (Formulations 1 and 2) containing comparable (Formulation 2) or higher (Formulation 1) concentration of sodium lauryl sulfate plus five times nonionic surface active agent (polysorbate 80 or polyoxyethylene(40) monostearate) showed little irritativeness. This indicates that the strong irritativeness of anionic surface active agent sodium lauryl sulfate was blocked by the addition of either of these nonionic surface active agents. At the same time, that these nonionic surface active agents, which has been known to those skilled in the art surely to exhibit clear irritativeness at such high concentrations, did not exhibit any irritativeness as shown in the above results (Formulations 1 and 2), indicates that the irritativeness of the nonionic surface active agents also was blocked by the addition of the anionic surface active agent. It has been totally unexpectable that the high concentrations of the two types of surface active agents, either of which must be highly irritative to the conjunctiva in the common knowledge of those skilled in the pertaining art, mutually blocked their irritativeness by combination.

### Anterior Ocular Irritation Test in Rabbit - 2

Using increased concentrations of surface active agents with varying their combinations, similar tests with the above were performed.

### Animals and topical application procedures:

The aqueous solutions of surface active agents as shown in Formulations 4 and 5 below were topically applied to the eyes of 8 to 9-week old Japanese white rabbits (Groups 4 and 5, respectively; three animals/group), and evaluated for the presence and extent of irritation to the anterior segment of the eye. For each formulation, topical application was carried out to one eye (tested eye) with two drops a time, three times at one hour intervals for Formulations 1 and 2. The opposite eye (control eye) simultaneously received the following Formulations for comparison 4C and 5C, which contained an anionic surface active agent alone.

| Formulation 4: | |
|---|---|
| Sodium lauryl sulfate | 1.0 W/V% |
| POE(25) lauryl ether | 5.0 W/V% |

| Formulation 4C: | |
|---|---|
| Sodium lauryl sulfate | 1.0 W/V% |

| Formulation 5: | |
|---|---|
| Sodium N-lauroyl sarcosinate | 1.0 W/V% |
| Decaglyceryl monolaurate | 5.0 W/V% |

| Formulation 5C: | |
|---|---|
| Sodium N-lauroyl sarcosinate | 1.0 W/V% |

### Method for Observation and Evaluation:

For each group, gross observation of the anterior segment of the eye was performed and evaluated according to modified Draize method immediately before the initiation of the topical applications, thirty minutes after each application, and 24 and 48 hours after the final application. Observation of fluorescein staining on the cornea was performed immediately before the initiation the topical applications, and thirty minutes and 24 hours after the final application.Results:

### (1) Gross Observation

The results are presented in Tables 7 to 10. For the cornea and iris, figures are omitted, for their scores were all "0".

**TABLE 7**

| Scores for Formulation 4 applied eyes of Group 4 | | | | | | |
|---|---|---|---|---|---|---|
| Observ. time | Ini. | 1st | 2nd | 3rd | 24hr | 48hr |
| Redness of palpebral conjunctiva | 0 | 0 | 0.17 | 0.17 | 0 | 0 |
| Edema of palpebral conjunctiva | 0 | 0 | 0 | 0 | 0 | 0 |
| Redness of bulbar conjunctiva | 0 | 0 | 0 | 0 | 0 | 0 |
| Nictitating membrane | 0 | 0 | 0 | 0 | 0 | 0 |
| Discharge | 0 | 0 | 0 | 0 | 0 | 0 |
| Total score | 0 | 0 | 0.17 | 0.17 | 0 | 0 |

**TABLE 8**

| Scores for Formulation 4C applied eyes of Group 4 | | | | | | |
|---|---|---|---|---|---|---|
| Observ. time | Ini. | 1st | 2nd | 3rd | 24hr | 48hr |
| Redness of palpebral conjunctiva | 0 | 1.33 | 1.67 | 1.67 | 0.67 | 0.17 |
| Edema of palpebral conjunctiva | 0 | 0.33 | 1.17 | 1.33 | 0 | 0 |
| Redness of bulbar conjunctiva | 0 | 0.83 | 1.33 | 1.50* | 0.50 | 0 |
| Nictitating membrane | 0 | 0.83 | 0.67 | 0.50 | 0.83 | 0.33 |
| Discharge | 0 | 0.33 | 1.00 | 1.67 | 0.33 | 0 |
| Total score | 0 | 3.66 | 5.83 | 6.67 | 2.33 | 0.50 |

| | | | | | | |
|---|---|---|---|---|---|---|
| * : Observation not possible in one eye out of the three due to edema of conjunctiva . | | | | | | |

**TABLE 9**

| Scores for Formulation 5 applied eyes of Group 5 | | | | | | |
|---|---|---|---|---|---|---|
| Observ. time | Ini. | 1st | 2nd | 3rd | 24hr | 48hr |
| Redness of palpebral conjunctiva | 0 | 0.83 | 0.83 | 0.50 | 0 | 0 |
| Edema of palpebral conjunctiva | 0 | 0 | 0 | 0.17 | 0 | 0 |
| Redness of bulbar conjunctiva | 0 | 0 | 0.33 | 0.33 | 0 | 0 |
| Nictitating membrane | 0 | 0 | 0 | 0 | 0 | 0 |
| Discharge | 0 | 0 | 0 | 0.67 | 0 | 0 |
| Total score | 0 | 0.83 | 1.17 | 1.67 | 0 | 0 |

**TABLE 10**

| Scores for Formulation 5C applied eyes of Group 5 | | | | | | |
|---|---|---|---|---|---|---|
| Observ. time | Ini. | 1st | 2nd | 3rd | 24hr | 48hr |
| Redness of palpebral conjunctiva | 0 | 1.33 | 1.33 | 1.00 | 1.00 | 0.33 |
| Edema of palpebral conjunctiva | 0 | 0.50 | 1.50 | 2.33 | 0 | 0 |
| Redness of bulbar conjunctiva | 0 | 1.00*¹ | 0.75 *² | - | 0.17 | 0 |
| Nictitating membrane | 0 | 0.17 | 0.17 | 0.83 | 0.83 | 0.17 |
| Discharge | 0 | 0.33 | 0.67 | 1.33 | 033 | 0 |
| Total score | 0 | 3.33 | 4.42 | 5.50 | 2.33 | 0.50 |

| | | | | | | |
|---|---|---|---|---|---|---|
| * 1: Observation not possible in one eye out of the three due to edema of conjunctiva | | | | | | |
| * 2: Observation not possible in two eyes out of the three due to edema of conjunctiva | | | | | | |
| - : Observation not possible in all of the three eyes due to edema of conjunctiva | | | | | | |

As evident from the tables, high degree of irritativeness was observed for all of the items in the table, in particular, severe edema of palpebral conjunctiva, for the comparison Formulation 4C (1% sodium lauryl sulfate alone) and Formulation 5C (1% Sarcosinate LN alone). In the eye with severe edema of palpebral conjunctiva, the edema covered the bulbar conjunctiva and thereby rendering it impossible to conduct evaluation of the redness of bulbar conjunctiva. Thus, evaluation of redness of bulbar conjunctiva was conducted only for eyes without severe edema. Therefore, the figures for redness of bulbar conjunctiva in Tables 8 and 10 are considered to be smaller than the degree of actually occurring redness. In contrast to these formulations for comparison, no irritativeness was observed with Formulation 4, with only mild, normally observable degree of redness of palpebral conjunctiva. With Formulation 5, though somewhat increased redness was seen, irritation was very weak, if any, and thus it was found that irritativeness is nearly totally eliminated when compared with the severe irritativeness of the comparison Formulation 5C.

### (2) Findings in corneal fluorescein staining:

With the comparison Formulations 4C and 5C, the number of diffuse stained spots remarkably increased, indicating occurrence of keratitis superficialis diffusa (KSD), and with the comparison Formulation 5C, in particular, a large geographic stained area was observed. In contrast, no abnormality was noted with Formulations 4 and 5.

### Conclusion:

As evident from these results, while the comparison Formulation 4C, which contains an anionic surface active agent sodium lauryl sulfate alone at 1%, and the comparison Formulation 5C containing an anionic surface active agent Sarcosinate LN also alone at 1%, exhibited high irritativeness, Formulations 4 and 5, which concurrently contain 5% POE(25) lauryl ether or 5% decaglyceryl monolaurate, respectively, showed little irritativeness, and, in particular, no irritativeness with Formulation 4. This again demonstrates that, as shown in the results of the preceeding Test-1, the combination of an anionic surface active agent and a nonionic surface active agent blocks the strong irritativeness of the anionic surface active agent and, at the same time, also blocks the irritativeness expected from the use of high concentrations of the nonionic surface active agent.

### Anterior Ocular Irritation Test in Rabbit - 3

### Animals and topical application procedures:

The compositions of formulations 6 and 6C below, which contain an ampholytic surface active agent lauryldimethylaminoacetic acid betaine (purchased as a 35 % aqueous solution under the name of AM-301) were used. One eye (tested eye) of male Japanese white rabbits received Formulation 6 and the opposite eye Formulation 6C (formulation for comparison) with two drops a time (about 100 µL), three times at one hour intervals.

Gross observation of the anterior segment of the eye was performed immediately before the initiation of the topical applications, thirty minutes after each of the first, and third applications, and on the following day. Slitlamp microscopy for fluorescein stained spots on the cornea was performed thirty minutes before the initiation of the applications, thirty minutes after the final application, and on the following day. For gross observation, scoring and evaluation was made according to the modified Draize test.

| Formulation 6: | |
|---|---|
| Lauryldimethylaminoacetic acid betaine | 0.5 g |
| Polysorbate 80 | 2.5 g |
| Distilled water | q.s. |
| Total amount | 100 mL |
| (pH 6.2, Osmotic pressure: 46 mOsm) | |

| Formulation 6C: | |
|---|---|
| Lauryldimethylaminoacetic acid betaine | 0.5 g |
| Distilled water | q.s. |
| Total amount | 100 mL |
| (pH 6.7, Osmotic pressure: 39 mOsm) | |

### Results:

### (1) Gross Observation of the Anterior Segment of the Eye

The results are presented in Tables 11 and 12.

**TABLE 11**

| Scores for Formulation 6 applied eyes | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0 | 0.5 | 0.5 | 0 |
| Edema of palpebral conjunctiva | 0 | 0 | 0 | 0.5 | 0 |
| Redness of bulbar conjunctiva | 0 | 0 | 0 | 0.5 | 0 |
| Nictitating membrane | 0 | 0 | 0.5 | 0 | 0 |
| Discharge | 0 | 0 | 0 | 1.0 | 0 |
| Total score | 0 | 0 | 1.0 | 2.5 | 0 |

**TABLE 12**

| Scores for Formulation 6C applied eyes | | | | | |
|---|---|---|---|---|---|
| Observ. time | Initial | 1st | 2nd | 3rd | Final |
| Degree of corneal opacity | 0 | 0 | 0 | 2.0 | 0 |
| Area of opacity | 0 | 0 | 0 | 2.0 | 0 |
| Iris | 0 | 0 | 0 | 0 | 0 |
| Redness of palpebral conjunctiva | 0 | 0.5 | 1.0 | 2.0 | 0 |
| Edema of palpebral conjunctiva | 0 | 0 | 1.0 | 3.0 | 0 |
| Redness of bulbar conjunctiva | 0 | 0.5 | 0.5 | - | 0 |
| Nictitating membrane | 0 | 0 | 0.5 | 1.0 | 0 |
| Discharge | 0 | 0 | 0 | 2.0 | 0 |
| Total score | 0 | 1.0 | 3.0 | 12.0 | 0 |

| | | | | | |
|---|---|---|---|---|---|
| - : Observation not possible due to edema | | | | | |

As evident from the tables, with Formulation 6C (ampholytic surface active agent lauryldimethylaminoacetic acid betaine alone), very strong irritation was observed such as redness of palpebral conjunctiva and nictitating membrane, edema of conjunctiva, discharge as well as corneal opacity. In the eyes with severe edema of palpebral conjunctiva, the edema covered the bulbar conjunctiva and thereby rendering it impossible to conduct evaluation of the redness of bulbar conjunctiva. Thus, evaluation of redness of bulbar conjunctiva was conducted only for eyes without severe edema. Therefore, the figures for redness of bulbar conjunctiva in Table 12 are considered to be smaller than the degree of actually occurring redness. With application of mixed solution of lauryldimethylaminoacetic acid betaine and polysorbate, in contrast, all what was noted were a slight redness of palpebral conjunctiva, bulbar conjunctiva and nictitating membrane and discharge.

### (2) Findings in corneal fluorescein staining:

KSD were observed with Formulation 6C, which is a solution of lauryldimethylaminoacetic acid betaine alone, whereas no abnormality was noted with Formulation 6, which is a mixed solution with polysorbate.

### Conclusion:

As evident from the results, a high degree of irritativeness was observed with Formulation 6C containing only a surface active agent having a anionic active group, i.e., ampholytic surface active agent lauryldimethylaminoacetic acid betaine, whereas only a slight irritation was observed with Formulation 6, which concurrently contained five times amount of a nonionic surface active agent, polysorbate 80. This indicates that the strong irritativeness of the ampholytic surface active agent lauryldimethylaminoacetic acid betaine was blocked by the addition of the nonionic surface active agent polysorbate 80. At the same time, that polysorbate 80 did not exhibit any irritativeness even at this high concentration indicates that the irritativeness of the nonionic surface active agent was also blocked by the addition of the ampholytic surface active agent.

### Anterior Ocular Irritation Test in Rabbit - 4

### Animals and topical application procedures:

Following formulations 7A, 7B, 7C, 8, 8C, 9 and 9C, which contain sodium lauryl sulfate as a surface active agent having an anionic active group, and distilled water were topically applied to the eye of male Japanese white rabbits with two drop a time (about 100 µL), three times at one hour intervals, and then the animals were examined.

Gross observation of the anterior segment of the eye was performed immediately before the initiation of the topical applications, thirty minutes after each of the first, and third applications, and on the following day. Slitlamp microscopy for fluorescein staining on the cornea was performed thirty minutes before the initiation of the applications, thirty minutes after the final application, and on the following day. For gross observation, modified Draize test was employed for scoring and evaluation.

| Formulation 7A: | |
|---|---|
| Sodium lauryl sulfate | 0.5 g |
| Polysorbate 80 | 0.5 g |
| Distilled water | q.s. |
| (pH 6.5) Total amount | 100 mL |

| Formulation 7B: | |
|---|---|
| Sodium lauryl sulfate | 0.5 g |
| Polysorbate 80 | 1.0 g |
| Distilled water | q.s. |
| (pH 6.6) Total amount | 100 mL |

| Formulation 7C: | |
|---|---|
| Sodium lauryl sulfate | 0.5 g |
| Distilled water | q.s. |
| (pH 6.0) Total amount | 100 mL |

| Formulation 8: | |
|---|---|
| Sodium lauryl sulfate | 0.1 g |
| Polysorbate 80 | 0.5 g |
| Distilled water | q.s. |
| (pH 6.7) Total amount | 100 mL |

| Formulation 8C: | |
|---|---|
| Sodium lauryl sulfate | 0.1 g |
| Distilled water | q.s. |
| (pH 5.9) Total amount | 100 mL |

| Formula 9: | |
|---|---|
| Sodium lauryl sulfate | 2.0 g |
| Polysorbate 80 | 10.0 g |
| Distilled water | q.s. |
| (pH 6.8) Total amount | 100 mL |

| Formulation 9C: | |
|---|---|
| Sodium lauryl sulfate | 2.0 g |
| Distilled water | q.s. |
| (pH 6.0) Total amount | 100 mL |

### Results:

### (1) The results are presented in Tables 13 to 20. In the titles to the tables, "SLS" denotes sodium lauryl sulfate, and "PS" polysorbate 80

**TABLE 13**

| Scores for eyes receiving Formulation 7A (0.5% SLS + 0.5% PS) | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0.75 | 0.75 | 1.25 | 0.25 |
| Edema of palpebral conjunctiva | 0 | 0 | 0 | 0 | 0 |
| Redness of bulbar conjunctiva | 0 | 0.75 | 0.75 | 1.50 | 0 |
| Nictitating membrane | 0 | 0 | 0.25 | 0.25 | 0.25 |
| Discharge | 0 | 0 | 0 | 1.00 | 0 |
| Total score | 0 | 1.50 | 1.75 | 4.00 | 0.50 |

**TABLE 14**

| Scores for eyes receiving Formulation 7B (0.5% SLS + 1.0% PS) | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0.50 | 0.75 | 0.50 | 0 |
| Edema of palpebral conjunctiva | 0 | 0 | 0 | 0 | 0 |
| Redness of bulbar conjunctiva | 0 | 0.25 | 0.25 | 0.75 | 0 |
| Nictitating membrane | 0 | 0 | 0 | 0 | 0 |
| Discharge | 0 | 0 | 0 | 0.50 | 0 |
| Total score | 0 | 0.75 | 1.00 | 1.75 | 0 |

**TABLE 15**

| Scores for eyes receiving Formulation 7C (0.5% SLS) | | | | | |
|---|---|---|---|---|---|
| Observ. time | Initial | 1st | 2nd | 3rd | Final |
| Degree pf corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 1.0 | 1.0 | 2.0 | 0 |
| Edema of palpebral conjunctiva | 0 | 0.5 | 0.5 | 1.0 | 0 |
| Redness of bulbar conjunctiva | 0 | 1.0 | 0.5 | 2.0 | 0 |
| Nictitating membrane | 0 | 0 | 0.5 | 2.0 | 0 |
| Discharge | 0 | 0 | 0 | 0 | 0 |
| Total score | 0 | 2.5 | 2.5 | 7.0 | 0 |

**TABLE 16**

| Scores for eyes receiving distilled water | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0 | 0 | 0 | 0 |
| Edema of palpebral conjunctiva | 0 | 0 | 0 | 0 | 0 |
| Redness of bulbar conjunctiva | 0 | 0 | 0 | 0 | 0 |
| Nictitating membrane | 0 | 0 | 0 | 0 | 0 |
| Discharge | 0 | 0 | 0 | 0 | 0 |
| Total score | 0 | 0 | 0 | 0 | 0 |

**TABLE 17**

| Scores for eyes receiving Formulation 8 (0.1% SLS + 0.5% PS) | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0 | 0 | 0 | 0 |
| Edema of palpebral conjunctiva | 0 | 0 | 0 | 0 | 0 |
| Redness of bulbar conjunctiva | 0 | 0 | 0 | 0 | 0 |
| Nictitating membrane | 0 | 0 | 0 | 0 | 0 |
| Discharge | 0 | 0 | 0 | 0 | 0 |
| Total score | 0 | 0 | 0 | 0 | 0 |

**TABLE 18**

| Scores for eyes receiving Formulation 8C (0.1% SLS) | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0 | 1.0 | 2.0 | 0 |
| Edema of palpebral conjunctiva | 0 | 0 | 0 | 0 | 0 |
| Redness of bulbar conjunctiva | 0 | 0 | 0 | 0.5 | 0 |
| Nictitating membrane | 0 | 0 | 0 | 0.5 | 0 |
| Discharge | 0 | 0 | 0 | 0 | 0 |
| Total score | 0 | 0 | 1.0 | 3.0 | 0 |

**TABLE 19**

| Scores for eyes receiving Formulation 9 (2.0% SLS + 10% PS) | | | | | |
|---|---|---|---|---|---|
| Observ. time | Initial | 1st | 2nd | 3rd | Final |
| Degree of corneal opacity | 0 | 0 | 0 | 0 | 0 |
| Area of opacity | 0 | 0 | 0 | 0 | 0 |
| Iris | 0 | 0 | 0 | 0 | 0 |
| Redness of palpebral conjunctiva | 0 | 0 | 0 | 1.0 | 0.5 |
| Edema of palpebral conjunctiva | 0 | 0 | 0 | 0 | 0 |
| Redness of bulbar conjunctiva | 0 | 0 | 0 | 0 | 0 |
| Nictitating membrane | 0 | 0 | 0 | 0 | 0 |
| Discharge | 0 | 0 | 0 | 0 | 0 |
| Total score | 0 | 0 | 0 | 1.0 | 0.5 |

**TABLE 20**

| Scores for eyes receiving Formulation 9C (2.0% SLS) | | | | | |
|---|---|---|---|---|---|
| Observ. time | Initial | 1st | 2nd | 3rd | Final |
| Degree of corneal opacity | 0 | 0 | 0 | 2.0 | 2.0 |
| Area of opacity | 0 | 0 | 0 | 3.0 | 1.0 |
| Iris | 0 | 0 | 0 | 0 | 0 |
| Redness of palpebral conjunctiva | 0 | 1.0 | 2.0 | 2.0 | 3.0 |
| Edema of palpebral conjunctiva | 0 | 0 | 1.0 | 2.0 | 0.5 |
| Redness of bulbar conjunctiva | 0 | 0.5 | 1.0 | - | 1.0 |
| Nictitating membrane | 0 | 0 | 0.5 | 1.0 | 2.0 |
| Discharge | 0 | 0 | 0 | 2.0 | 0 |
| Total score | 0 | 1.5 | 4.5 | 13.0 | 8.5 |

| | | | | | |
|---|---|---|---|---|---|
| - : Observation not possible due to edema | | | | | |

As shown in Table 15, overall strong irritation, in particular with conjunctival edema, was observed with 0.5 % sodium lauryl sulfate solution (Formulation 7C), and the total score after the third application reached 7.0. In the eyes with severe edema of palpebral conjunctiva, the edema covered the bulbar conjunctiva and thereby rendering it impossible to conduct evaluation of the redness of bulbar conjunctiva. Thus, evaluation of redness of bulbar conjunctiva was conducted only for eyes without severe edema. Therefore, the figures for redness of bulbar conjunctiva in Table 20 are considered to be smaller than the degree of actually occurring redness. In contrast, with Formulation 7B, in which 1.0 % polysorbate 80 is additionally contained (anionic surface active agent : nonionic surface active agent = 1:2) shown in Table 14, there were observed generally much less irritation, no conjunctival edema in particular, and thus total score after the third application was remarkably suppressed to 1.75. In addition, even for the solution of 0.5 % sodium lauryl sulfate + 0.5 % polysorbate 80 (Formulation 7B; anionic surface active agent : nonionic surface active agent = 1:1), irritation was generally found to be less than Formulation 7C with no conjunctival edema observed, and total score after the third application was clearly suppressed to 4.00, when compared with the figure 7.0 with Formula 7C. Topical application of distilled water showed no irritation (Table 16). It is known from these results that there is a irritation suppressive effect when the ratio of sodium lauryl sulfate (an anionic surface active agent) to polysorbate 80 (a nonionic surface active agent) is 1:1, and that the suppressive effect is more remarkable when the ratio is 1: 2.

Moreover, for the lower concentration ranges of surface active agents, comparison of the total score obtained with 0.1 % sodium lauryl sulfate solution (Formulation 8C) in Table 18 with those obtained with 0.1 % sodium lauryl sulfate + 0.5 % polysorbate 80 (Formulation 8: anionic surface active agent : nonionic surface active agent = 1:5) shown in Table 17 shows that a slight irritation noted with the former was not observable with the latter. This indicates that an anionic surface active agent, sodium lauryl sulfate, somewhat exhibits irritation even at this low concentration, but that suppression of irritation is obtainable, even at such concentration, by combination with a nonionic surface active agent, polysorbate 80.

Moreover, for the higher concentration ranges of surface active agents, comparison of the scores obtained with 2.0 % sodium lauryl sulfate (Formulation 9C) shown in Table 20 with those obtained with 2.0 % sodium lauryl sulfate + 10 % polysorbate 80 (Formulation 9: anionic surface active agent : nonionic surface active agent = 1:5) shown in Table 19 shows that the severe irritation with the former was substantially completely suppressed with the latter.

### (2) Findings in corneal fluorescein staining:

While KSD covering the overall cornea were observed with Formulation 7C containing 0.5 % sodium lauryl sulfate alone (Table 15), stained spots were only noted in limited areas with Formulations 7A and 7B added with polysorbate at respective concentration ratios of 1:1 to 1:2 (Tables 13 and 14), indicating suppression of irritation to the cornea. No abnormality was observed with low concentration formulations 8 (Table 14) and 8C (Table 18). While corneal erosion was noted in wide areas with formulation 9C containing 2.0 % sodium lauryl sulfate alone (Table 20), which were heavily stained with fluorescein, no abnormality was noted in the cornea with formulation 9, which was added with polysorbate 80 at a concentration ratio of 1:5 (Table 19), indicating total suppression of irritation to the cornea.

### Conclusion:

The above results indicates that reduction of irritativeness of anionic surface active agents is observed even when the concentration ratio of anionic surface active agent to the nonionic surface active agents is 1:1, and that this irritation suppressive effect becomes more remarkable with increased proportion of the nonionic surface active agent. With regard to the concentration of surface active agents, it is shown that a slight irritativeness seen in a low concentration, i.e. 0.1 %, of the anionic surface active agent can be suppressed by addition of the nonionic surface active agent, and that a high degree of irritativeness of a high concentration, i.e. 2.0 %, of the anionic surface active agent can also be completely suppressed by addition of the nonionic surface active agent. Moreover, it also is shown that combination of the both types of surface active agents suppresses irritativeness expected with the use of high concentrations of a nonionic surface active agent alone.

### EMBODIMENT OF THE INVENTION

In practicing the present invention, the combination of surface active agents according to the present invention is employed instead of conventional compositions of surface active agents during the production of aqueous compositions which are, or have possibility to be, brought into contact with mucous membranes, such as external-use aqueous pharmaceutical preparations, e.g., eye drops, toothpaste, shampoo, contact lens care preparation (e.g., contact lens cleaner).

Moreover, the combination of surface active agents according to the present invention is employed instead of conventional surface active agents, in solubilizing, or homogeneously dispersing in the form of emulsion of suspension, insoluble of scarcely soluble drugs in water in order to produce external-use non-irritative aqueous pharmaceutical preparations applicable to sensitive tissues such as mucous membranes.

In these cases, one or more surface active agents may be used either for surface active agents having an anionic active group or nonionic surface active agents. The concentration (W/V %) ratio of surface active agents having an anionic active group to nonionic surface active agents is preferably 1:1 to 1:6, more preferable 1:2 to 1:6, and still more preferably 1:4 to 1:6.

The concentration ranges of surface active agents according to the present invention are: in general, preferably 0.1 to 2 W/V%, more preferably 0.1 to 1 W/V%, and still more preferably 0.1 to 0.5 W/V% for surface active agents having an anionic active group with a proviso that the above identified mutual ratio is maintained, and, in general, preferably 0.1 to 10 W/V%, more preferably 0.2 to 5 W/V%, and still more preferably 0.5 to 2.5 W/V% for nonionic surface active agents.

Examples of drugs which may be provided in the form of external-use aqueous pharmaceutical preparation include sulfa drugs, e.g., sulfadiazine, sulfisoxazole, sulfisomidine, sulfadimetoxine, sulfamethoxypridazine, sulfamethoxazole, sulfaethidole, sulfamethomidine, sulfaphenazole, sulfaguanidine, phthalylsulfathiazole, succinylsulfathiazole and their salts. The invention is also applicable to other insoluble or scarcely soluble drugs such as steroids, e.g., fluorometholone, as well as pharmaceutical ingredients which are soluble at unphysiologic pH but insoluble at physiologically acceptable pH.

For dissolving or homogeneously dispersing insoluble or scarcely soluble ingredients in water for the purpose of obtaining external-use aqueous pharmaceutical preparations according to the present invention, combined surface active agents having an anionic active group and nonionic surface active agents are used, instead of the conventional, sole use of nonionic surface active agents, following the same procedure well known to those skilled in the art when they employ nonionic surface active agents. Concentrations of surface active agents to be used may be determined according to routine considerations by those skilled in the art in preparing pharmaceutical preparations, such as the properties and amount of the pharmaceutical ingredient intended to dissolve or homogeneously disperse. Even for pharmaceutical preparations for direct application to the mucous membrane in the eye, as far as they are not intended to be frequently applied, those combinations can be used without substantially causing irritation which contain up to 2 W/V% of surface active agents having an anionic active group and up to 10 W/V% of nonionic surface active agents. Other known ingredients to those skilled in the art which are conventionally used in preparing aqueous pharmaceutical preparations may also be used, e.g., pH adjusting agents, buffering agents, osmotic pressure adjusting agents, suspension aids, antioxidants, preservatives and the like.

### EXAMPLES

Embodiments of application of the present invention for solubilization of scarcely soluble drugs are presented below. Sulfioxazole and lidocaine, pharmacologically active compounds, are both scarcely soluble at pH 7, a pH close to the physiological pH desirable for eye drops and nasal drops.

### Example 1 Eye Drops

The following ingredients were admixed and stirred to dissolve sulfioxazole which is scarcely soluble inherently, filter-sterilized by a conventional method and filled into a dropper container to obtain eye drops.

| | |
|---|---|
| Sulfisoxazole | 0.05 g |
| Sodium lauryl sulfate | 0.6 g |
| Polyoxyethylene lauryl ether (BL-25) | 3.0 g |
| Boric acid | 1.7 g |
| Benzalkonium chloride | 0.01 g |
| Sodium hydroxide | q.s. (pH 7.0) |
| Purified water | q.s. |
| Total amount | 100 mL |

### Example 2 Nasal Drops

The following ingredients were admixed and stirred to dissolve lidocaine which is scarcely soluble at this pH, filtered by a conventional method and filled into a dropper container to obtain nasal drops.

| | |
|---|---|
| Lidocaine | 0.5 g |
| Sodium N-lauroyl sarcosinate | 0.6 g |
| Decaglyceryl monolaurate (Decaglyn 1-L) | 3.0 g |
| Conc. glycerol | 1.6 g |
| Benzalkonium chloride | 0.01 g |
| Hydrochloric acid | q.s. (pH 7.0) |
| Purified water | q.s. |
| | 100 mL |

### Example 3 Eye Drops

The following ingredients were admixed and stirred to dissolve sulfioxazole which is scarcely soluble inherently, filter-sterilized by a conventional method and filled into a dropper container to obtain eye drops.

| | |
|---|---|
| Sulfisoxazole | 0.05 g |
| Sodium lauryl sulfate | 0.6 g |
| Polyoxyethylene lauryl ether (BL-25) | 3.0 g |
| Boric acid | 1.7 g |
| Sodium hydroxide | q.s. (pH 7.0) |
| Purified water | q.s. |
| Total amount | 100 mL |

### Example 4 Nasal Drops

The following ingredients were admixed and stirred to dissolve lidocaine which is scarcely soluble at this pH, filtered by a conventional method and filled into a dropper container to obtain nasal drops.

| | |
|---|---|
| Lidocaine | 0.5 g |
| Sodium N-lauroyl sarcosinate (Sarcosinate LN) | 0.6 g |
| Decaglyceryl monolaurate (Decaglyn 1-L) | 3.0 g |
| Conc. glycerol | 1.6 g |
| Hydrochloric acid | q.s. (pH 7.0) |
| Purified water | q.s. |
| Total amount | 100 mL |

### Example 5 Eye Drops

The following ingredients were aseptically admixed and stirred, giving a stable suspension, which were filled into a dropper container to obtain eye drops (osmotic pressure: 307 mOsmol/kg).

| | |
|---|---|
| Fluorometholone | 0.1 g |
| Sodium N-lauroyl sarcosinate | 0.5 g |
| Decaglyceryl monolaurate | 2.5 g |
| Boric acid | 1.6 g |
| Benzalkonium chloride | 0.01 g |
| Sodium hydroxide | q.s. (pH 7.0) |
| Sterile purified water | q.s. |
| Total amount | 100 mL |

### Example 6 Shampoo

The following ingredients were admixed and stirred by a conventional method to obtain a shampoo (a clear and colorless, viscous liquid; pH 10.1). NB: For polyoxyethylene(3)alkyl ether sulfate sodium salt, 17.78 g of its 27 W/V% aqueous solution was used.

| | |
|---|---|
| Sodium polyoxyethylene(3)alkyl ether sulfate | 4.8 g |
| Lauric acid diethanolamide | 24.0 g |
| Propylene glycol | 2.0 g |
| Methyl p-hydroxybenzoate | 0.01 g |
| Purified water | q.s. |
| Total amount | 100 mL |

### Example 7 Toothpaste

The following ingredients were admixed by a conventional method and well kneaded to obtain a toothpaste (a white paste; pH 8.9).

| | |
|---|---|
| Calcium chloride | 39.0 g |
| Sorbitol | 22.0 g |
| Carmellose sodium | 1.1 g |
| Sodium lauryl sulfate | 1.0 g |
| Polysorbate 80 | 5.0 g |
| Saccharin sodium | 0.1 g |
| Methyl p-hydroxybenzoate | 0.01 g |
| Purified water | q.s. |
| Total amount | 100 g |

### Example 8 Contact Lens Cleaner

The following ingredients were admixed and stirred by a conventional method to obtain a contact lens cleaner for hard contact lenses and oxygen permeable contact lenses (clear and colorless liquid; pH 6.0). (N.B: For triethanolamine N-lauroyl-L-glutamate, 3.33 g of its 30 W/V% product was used.)

| | |
|---|---|
| Bioprase | 48000 PUN |
| Triethanolamine N-lauroyl-L-glutamate | 1.00 g |
| Polyoxyl 40 stearate | 5.0 g |
| Boric acid | 0.5 g |
| Disodium edetate | 0.1 g |
| Sodium hydroxide | q.s. (pH 6.0) |
| Purified water | q.s. |
| Total amount | 100 mL |

## Claims

1. A method of reducing irritativeness caused by surface active agents contained in a aqueous composition, by employing, in the production of said composition, in combination one or more surface active agents having an anionic active group with one or more nonionic surface active agents, wherein the mutual ratio of said one or more surface active agents having an anionic active group to said one or more nonionic surface active agents is 1:1 to 1:6 in concentration by weight.

2. The method of claim 1, wherein said one or more surface active agents having an anionic active group are contained at a concentration of 0.1 to 2 W/V%, and wherein said one or more nonionic surface active agents are contained at a concentration of 0.1 to 10 W/V%.

3. The method of claim 1 or 2, wherein said one or more surface active agents having an anionic active group are anionic surface active agents.

4. The method of Claim 1 or 2, wherein said one of more surface active agents having a anionic active group are ampholytic surface active agents.

5. An aqueous composition containing one or more surface active agents having an anionic active group together with one or more nonionic surface active agents, wherein the mutual ratio of said one or more surface active agents having an anionic active group to said one or more nonionic surface active agents is 1:1 to 1:6 in concentration by weight.

6. The aqueous composition of claim 5, wherein said one or more surface active agents having an anionic active group are contained at a concentration of 0.1 to 2 W/V%, and wherein said one or more nonionic surface active agents are contained at a concentration of 0.1 to 10 W/V%.

7. The aqueous composition of claim 5 or 6, wherein said composition further contains a pharmacologically active ingredient.

8. The aqueous composition of one of claims 5 to 7, wherein said composition is an external-use aqueous pharmaceutical preparation.

9. The aqueous composition of claim 8, wherein said composition is applicable to mucous membranes or the cornea.

10. The aqueous composition of claim 8, wherein said composition is in the form of eye drops, nasal drops, ear drops, dermatologic preparation, shampoo, toothpaste or contact lens care preparation.

11. The aqueous composition of one of claims 5 to 10, wherein said one or more surface active agents having an anionic active group are anionic surface active agents.

12. The aqueous composition of one of claims 5 to 10, wherein said one or more surface active agents having an anionic active group are ampholytic surface active agents.

13. A method of solubilization of an insoluble or scarcely soluble pharmaceutical ingredient in producing an external-use aqueous pharmaceutical preparation, comprising the step of mixing said pharmaceutical ingredient with an aqueous solution containing one or more surface active agents having an anionic active group and one or more nonionic surface active agents, wherein the mutual ratio of said one or more surface active agents having an anionic active group to said one or more nonionic surface active agents is 1:1 to 1:6 in concentration by weight.

14. The method of claim 13, wherein said one or more surface active agents having an anionic active group are contained at a concentration of 0.1 to 2 W/V%, and wherein said one or more nonionic surface active agents are contained at a concentration of 0.1 to 10 W/V%.

15. The method of claim 13, wherein said external-use aqueous pharmaceutical preparation is in the form of eye drops, nasal drops, ear drops, or dermatologic preparation.

16. The method of one of claims 13 to 15, wherein said one or more surface active agents having an anionic active group are anionic surface active agents.

17. The method of one of claims 13 to 15, wherein said one or more surface active agents having an anionic active group are ampholytic surface active agents.
